# EUROPEAN PATENT SPECIFICATION

(11) **EP 2 943 282 B1**
(45) Date of publication and mention of the grant of the patent: **08.11.2017**
(21) Application number: 14702676.9
(22) Date of filing: 08.01.2014
(51) Int. Cl.: B01L 3/00, A61M 1/16

(54) **SYSTEMS AND METHODS FOR INCREASING CONVECTIVE CLEARANCE OF UNDESIRED PARTICLES IN A MICROFLUIDIC DEVICE**
SYSTEME UND METHODEN ZUR STEIGERUNG DER KONVEKTIVEN CLEARANCE VON UNERWÜNSCHTEN PARTIKELN IN EINER MIKROFLUIDISCHEN VORRICHTUNG
SYSTEMES ET PROCEDES POUR AUGMENTER LA CLEARANCE PAR CONVEXION DES PARTICULES INDÉSIRABLES DANS UN DISPOSITIF MICROFLUIDIQUE

(30) Priority: 11.01.2013 US 201313739701
(43) Date of publication of application: 18.11.2015
(73) Proprietor: The Charles Stark Draper Laboratory, Inc., Cambridge, MA 02139 (US)
(72) Inventor: HARJES, Daniel I., Acton, Massachusetts 01720 (US); DIBIASIO, Christopher, Stoughton, Massachusetts 02072 (US); CHAREST, Joseph L., Cambridge, Massachusetts 02138 (US); FINLEY, Violet, Medford, Massachusetts 02155 (US); SOONG, Ricky, Cambridge, Massachusetts 02140 (US); BORENSTEIN, Jeffrey T., Newton, Massachusetts 02464 (US)
(74) Representative: Hanna Moore + Curley
(86) International application number: PCT/US2014/010684
(87) International publication number: WO 2014/110133

(56) References cited:
- US-A1- 2008 318 324
- US-A1- 2011 082 563
- VIRGINIA VANDELINDER ET AL: "Perfusion in Microfluidic Cross-flow: Separation of white Blood Cells from Whole Blood and Exchange of Medium in a Continuous Flow", ANALYTICAL CHEMISTRY, vol. 79, no. 5, 1 March 2007 (2007-03-01), pages 2023-2030, XP002722221,

## Description

### BACKGROUND

A dialysis device contains a series of fluid channels separated by a permeable membrane. Convective clearance of solutes from blood in the device is determined by the transmembrane pressure in the device. Typically, the transmembrane pressure varies linearly along the length of the channels. Increasing the convective clearance in such devices involves increasing the length of the channels, which results in decreased patient mobility because of the need for a larger overall device size. Therefore, it is desirable to increase the amount of convective clearance within a compact dialysis device.
US2011/082563 discloses a microfluidic bioreactor device with intercommunicating micro-channels defined in two polymer layers separated by a membrane. A geometric parameter associated with the micro-channels in one layer may vary along a length of those channels.

### SUMMARY OF THE INVENTION

Aspects and implementations of the present disclosure are directed to a device for increasing convective transport of solutes in blood within a dialysis system.

At least one aspect is directed to a microfluidic device. The microfluidic device includes a first network of channels having a plurality of First Channels. Each First Channel has a height in the range of about 50 microns to about 500 microns, a width in the range of about 50 microns to about 900 microns, and a length in the range of about 3 centimeters to about 20 centimeters. The microfluidic device includes a second network of channels having at least one Second Channel complementary to one or more of the First Channels. The at least one Second Channel includes at least one pressurizing feature configured to yield a high pressure in a portion of the at least one Second Channel upstream from the pressurizing feature and a low pressure in a portion of the at least one Second Channel downstream from the pressurizing feature. The microfluidic device includes a filtration membrane separating the one or more First Channels from the at least one Second Channel. An upstream portion of the at least one Second Channel is located opposite a downstream portion of the one or more complementary First Channels, such that when fluid is flowed through the First and Second Channels, a non-linear pressure profile exists along the length of the at least one Second Channel and a pressure gradient exists across the membrane separating the one or more First Channels from the at least one complimentary Second Channel.

In some implementations, the non-linear pressure profile along the length of the at least one Second Channel is substantially a step function. The pressurizing feature can include a partial wall configured to restrict fluid flow in the at least one Second Channel, yielding the high pressure at an upstream portion of the at least one Second Channel and the low pressure at a downstream portion of the at least one Second Channel. The pressurizing feature can also include a section of the at least one Second Channel that tapers to reduce the cross-sectional area of a portion of the channel, yielding the high pressure at an upstream portion of the at least one Second Channel and the low pressure at a downstream portion of the at least one Second Channel. The pressurizing feature can also be a section of the at least one Second Channel configured to direct fluid along a circuitous path, yielding the high pressure at an upstream portion of the at least one Second Channel and the low pressure at a downstream portion of the at least one Second Channel. The pressurizing feature can also be a porous plug, a second membrane, or a gel inserted into the at least one Second Channel, yielding the high pressure at an upstream portion of the at least one Second Channel and the low pressure at a downstream portion of the at least one Second Channel. In some implementations, the pressurizing feature can be any device that extracts work from a flow of fluid, such as reverse pump or a turbine located inside the at least one Second Channel, yielding the high pressure at an upstream portion of the at least one Second Channel and the low pressure at a downstream portion of the at least one Second Channel. The pressurizing feature can also be a throttling device, such as a valve coupled to the at least one Second Channel, yielding the high pressure at an upstream portion of the at least one Second Channel and the low pressure at a downstream portion of the at least one Second Channel. The one or more First Channels can also include at least one second pressurizing feature.

In some implementations, the pressurizing feature is configurable to provide a desired pressure profile in the at least one Second Channel. The microfluidic device can also include a flow rate sensor and a pressure sensor for determining a flow rate and a fluid pressure in the one or more First Channels and the at least one Second Channel. The microfluidic device can also include a processor configured to control the pressurizing feature responsive to the determined flow rate or fluid pressure. In some implementations, the microfluidic device is configured to maintain a maximum pressure difference in the at least one Second Channel in a range of about 200 mmHg to about 2000 mmHg.

In some implementations, the membrane has a thickness in the range of about 5 µm to about 300 µm.. The membrane can be selected to allow clearance of particles with a molecular weight of no more than about 60 kDa.

In some implementations, the microfluidic device can include an anticoagulant coating on the inner surfaces of the one or more First Channels. The one or more First Channels can also be configured to maintain wall shear rates in the range of about 200 inverse seconds to about 2000 inverse seconds when blood is transported through the one or more First Channels. In some implementations, the one or more First Channels and the at least one Second Channel are configured for flowing fluid in opposite directions.

At least one aspect is directed to a method for filtering a first liquid containing an analyte to provide a filtered liquid containing less analyte than the first liquid. The method includes the step of introducing the first liquid into an inlet of a network of channels having one or more First Channels, each First Channel having a height in the range of about 50 microns to about 500 microns, a width in the range of about 50 microns to about 900 microns, and a length in the range of about 3 centimeters to about 20 centimeters. The method includes the step of introducing filtrate into an inlet of at least one Second Channel complementary to the one or more First Channels. The method includes the step of flowing the filtrate through at least one pressurizing feature in the at least one Second Channel. The pressurizing feature is configured to yield a high pressure in a portion of the at least one Second Channel upstream from the pressurizing feature, a low pressure in a portion of the at least one Second Channel downstream from the pressurizing feature, and a non-linear pressure profile along the length of the at least one Second Channel, such that at least some of the analyte of the first liquid is transported from the one or more First Channels through a membrane and into the at least one Second Channel. The method also includes the step of collecting the filtered liquid from an outlet of one or more of the First Channels. The outlet of the one or more First Channels is located opposite the inlet of the at least one corresponding Second Channel, and an inlet of the one or more First Channels is located opposite an outlet of the at least one complimentary Second Channel.

In some implementations, flowing the filtrate through the at least one pressurizing feature yields a pressure profile along the length of the at least one Second Channel that is substantially a step function. In some implementations, the method includes the step of determining at least one of a flow rate and a pressure in the one or more First Channels and the at least one Second Channel. The method can also include the step of adjusting the pressurizing feature, the flow rate in the one or more First Channels, or the flow rate in the at least one Second Channel, in response to the determinations of flow rate and pressure.

In some implementations, the step of introducing the first liquid includes introducing blood. The blood can be extracted from a patient and filtered blood can be returned to the patient. In some implementations, the one or more First Channels include an anticoagulant coating on its walls.

### BRIEF DESCRIPTION OF THE DRAWINGS

The accompanying drawings are not intended to be drawn to scale. Like reference numbers and designations in the various drawings indicate like elements. For purposes of clarity, not every component may be labeled in every drawing.
Figure 1A is a depiction of a microfluidic convective clearance device according to an illustrative implementation.
Figure 1B is a depiction of a blood substrate layer suitable for use in the microfluidic convective clearance device of Figure 1A, according to an illustrative implementation.
Figure 2 is a depiction of a first channel bilayer configuration suitable for use in the microfluidic device of Figure 1A, according to an illustrative implementation.
Figure 3 is a graph of the fluid pressure along the channels of the bilayer configuration depicted in Figure 2.
Figure 4 is a depiction of an alternative channel bilayer configuration suitable for use in the microfluidic device of Figure 1A, according to an illustrative implementation.
Figure 5 is a graph of the fluid pressure along the channels of the bilayer configuration depicted in Figure 4.
Figure 6 is a depiction of an alternative channel bilayer configuration suitable for use in the microfluidic device of Figure 1A, according to an illustrative implementation.
Figure 7 is a depiction of an alternative channel bilayer configuration suitable for use in the microfluidic device of Figure 1A, according to an illustrative implementation.
Figure 8 is a depiction of a system for cleansing a fluid incorporating the microfluidic convective clearance device of Figure 1A, according to an illustrative implementation.
Figure 9A is a depiction of an alternative channel bilayer configuration suitable for use in the fluid cleansing system of Figure 8 in a first state, according to an illustrative implementation.
Figure 9B is a depiction of the alternative channel bilayer configuration of Figure 9A in a second state, according to an illustrative implementation.
Figure 10 is a depiction of an alternative channel bilayer configuration suitable for use in the fluid cleansing system of Figure 1A, according to an illustrative implementation.
Figure 11 is a flow diagram of a method for filtering liquid containing an analyte, according to an illustrative implementation.

### DESCRIPTION OF CERTAIN ILLUSTRATIVE IMPLEMENTATIONS

Following below are more detailed descriptions of various concepts related to, and implementations of, a device for increasing convective transport of solutes in blood within a dialysis system. The various concepts introduced above and discussed in greater detail below may be implemented in any of numerous ways, as the described concepts are not limited to any particular manner of implementation. Examples of specific implementations and applications are provided primarily for illustrative purposes.

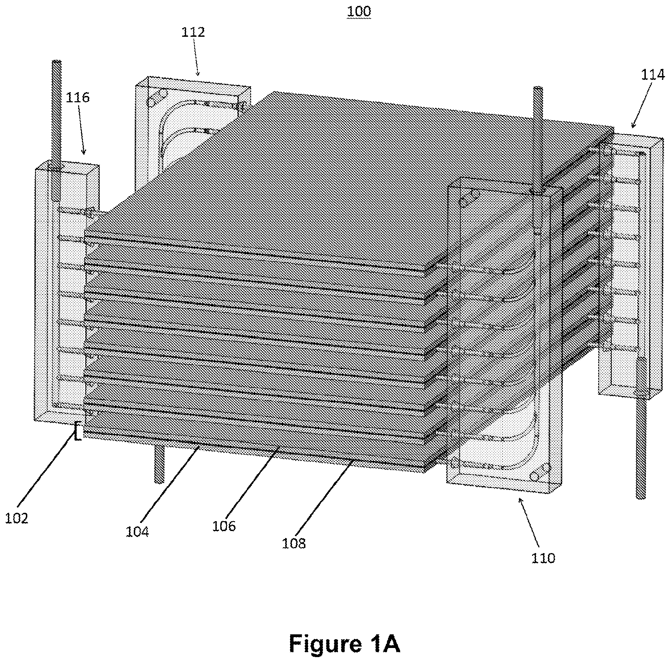

Figure 1A is a depiction of a microfluidic convective clearance device 100. The device 100 is composed of eight bilayers, as exemplified by the bilayer 102. Each bilayer 102 consists of a blood substrate layer, such as the blood substrate layer 104, and a filtrate substrate layer, such as the filtrate substrate layer 106, separated by a permeable membrane, such as the permeable membrane 108. A network of channels within the blood substrate layer 104 and the filtrate substrate layer 106 allows fluid (i.e. blood or filtrate) to be transported. The microfluidic convective clearance device 100 also includes a blood inlet manifold 110 and a blood outlet manifold 112, both coupled to the blood substrate layer 104. Similarly, a filtrate inlet manifold 114 and a filtrate outlet manifold 116 are coupled to the filtrate substrate layer 106. Blood enters the blood substrate layer 104 through the blood inlet manifold 110 and exits through the blood outlet manifold 112. Filtrate enters the filtrate substrate layer 106 through the filtrate inlet manifold 114 and exits through the filtrate outlet manifold 116.

Each bilayer 102 is parallel to each other bilayer 102. The blood substrate layer 104 and the filtrate substrate layer 106 each have a thickness in the range of about 10 microns to about 10 millimeters, and the membrane 108 has thickness in the range of about 500 nanometers to about 1 millimeter. In some implementations, adjacent bilayers 102 can be in contact with one another. In other implementations, the bilayers 102 can be separated by a distance of about 500 microns or more, as shown in Figure 1A.

The device 100 is designed for use in hemofiltration. The network of channels within the blood substrate layer 104 and the filtrate substrate layer 106 divide the fluid (i.e. blood and filtrate) so that a relatively large surface area of each fluid is exposed to the permeable membrane 108. Each channel of the blood substrate layer 104 is aligned with a corresponding channel of the filtrate substrate layer 106, so that the corresponding channels are separated by the permeable membrane 108. As the blood moves through the channels of the blood substrate layer 104, filtrate moves in the opposite direction through the filtrate substrate layer 106 and waste products and water are removed from the blood via diffusion and convection through the permeable membrane 108 into the filtrate substrate layer 106. Healthy blood remains in the blood substrate layer 104 and can then be recirculated into the body of a patient.

The blood substrate layer 104 and the filtrate substrate layer 106 can be made of a thermoplastic, such as polystyrene, polycarbonate, polyimide, or cyclic olefin copolymer (COC), biodegradable polyesters, such as polycaprolactone (PCL), or soft elastomers such as polyglycerol sebacate (PGS). The substrate layers 104 and 106 may alternatively be made of polydimethylsiloxane (PDMS), poly(N-isopropylacrylamide), or nanotubes or nanowires formed from, for example, carbon or zinc oxide. The substrates 104 and 106 are made of an insulating material to maintain temperature stability. In some implementations, the channels can be coated with cytophilic or cytophobic materials to promote or prevent the growth of cells, such as vascular endothelial cells, in the channels. The channels in the blood substrate layer 104 may also be coated with an anticoagulant to help prevent clotting of the blood in the blood substrate layer 104.

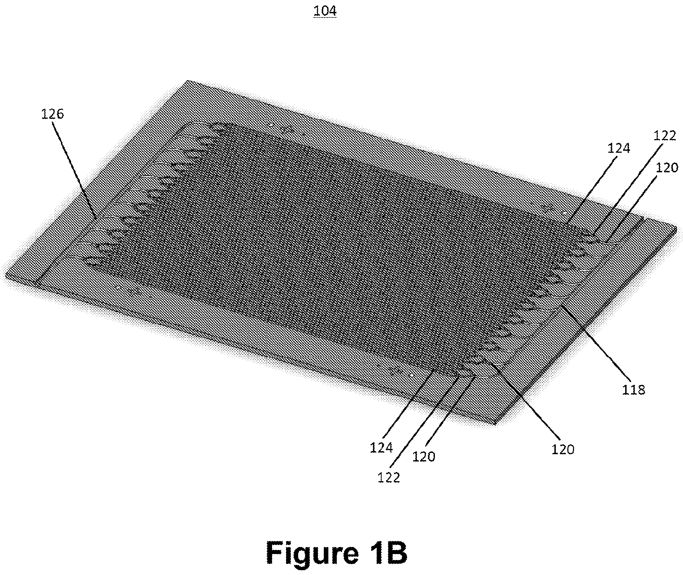

Figure 1B is a depiction of a blood substrate layer 104 suitable for use in the microfluidic convective clearance device of Figure 1A. The blood substrate layer 104 allows blood to be distributed across a relatively large surface area within the device 100. The blood substrate layer 104 has a network of channels, which includes a primary channel 118, secondary channels such as channel 120, tertiary channels such as channel 122, quaternary channels such as channel 124, and an outlet channel 126.

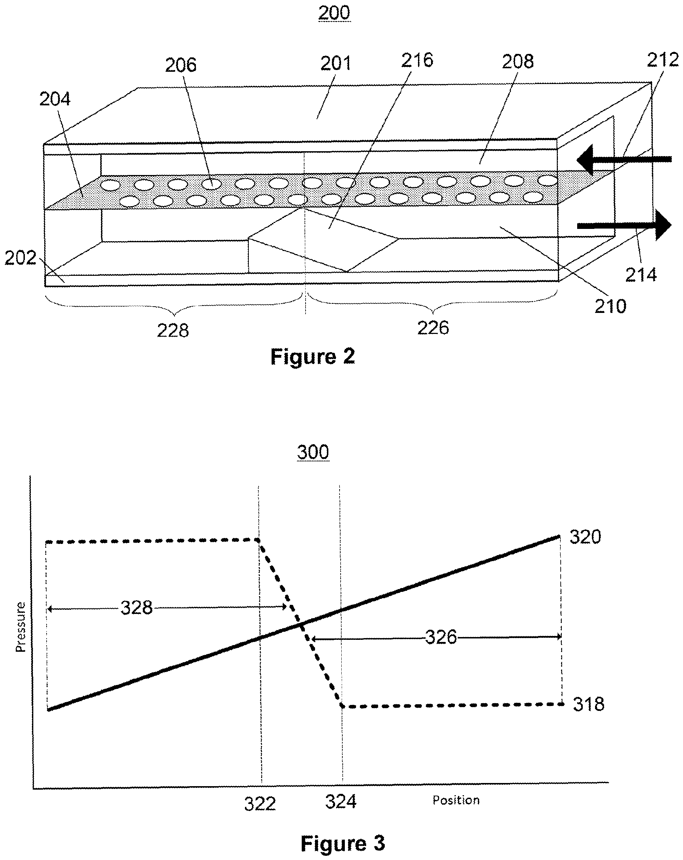

Figure 2 is a depiction of a first channel bilayer configuration 200 suitable for use in the microfluidic convective clearance device of Figure 1A. A blood substrate layer 201 and a filtrate substrate layer 202 are separated by a permeable membrane 204 for use as the blood substrate layer 104, the filtrate substrate layer 106, and the membrane 108, respectively, of the device 100 depicted in Figure 1A. The permeable membrane 204 includes pores 206. In some implementations, the membrane 204 has a thickness in the range of about 5 microns to about 300 microns and the pores 206 are sized to allow clearance of particles having a molecular weight of no more than about 60 kDa. In some other implementations, the membranes can have a thickness in the range of about 10 microns to about 200 microns, about 20 microns to about 100 microns, or about 30 microns to about 50 microns. In one implementation, the membrane is a 6 micron thick track etched membrane. The blood substrate layer 201 includes a blood flow channel 208 and the filtrate substrate layer 202 includes a filtrate channel 210. Arrows 212 and 214 show the direction of fluid flow in the blood flow channel 208 and filtrate channel 210, respectively. Although arrows 212 and 210 show fluid flowing in opposite directions in the blood channel 208 and the filtrate channel 210, in some implementations fluid in the blood channel 208 can flow in the same direction as fluid in the filtrate channel 210.

The filtrate channel 210 also includes a ramp 216. The ramp 216 is positioned on the bottom surface of the filtrate channel 210 and is configured to decrease the cross-sectional area of the filtrate channel 210 over a relatively small portion of the channel's length. Fluid flow is restricted by the decreased cross-sectional area, resulting in a high pressure upstream from the ramp 216 (towards the left-hand side of the filtrate channel 210 in Figure 2) and a lower pressure downstream from the ramp 216 (towards the right-hand side of the filtrate channel 210). Because the ramp 216 occupies only a small portion of the filtrate channel 210, the pressure decreases rapidly from the upstream portion of the filtrate channel 210 to the downstream portion of the filtrate channel 210.

Figure 3 is a graph 300 of the fluid pressure along the channels of the bilayer configuration depicted in Figure 2. The line 318 represents the fluid pressure in the filtrate channel 210 and the line 320 represents the fluid pressure in the blood flow channel 208. The ramp 216 lies along the bottom of the filtrate channel 210 between the points labeled 322 and 324 on the position axis of Figure 3. As shown, the ramp 216 creates a non-linear pressure profile along the length of the filtrate channel 210. To the left of point 322, the fluid pressure in filtrate channel 210 is high and relatively constant, due to the flow restriction imposed by the ramp 216. As fluid flows over the ramp 216, the pressure decreases until it reaches a minimum level at the end of the ramp 216, where the filtrate channel 210 has a relatively wide cross section.

The size and shape of the ramp 216 can be selected in order to achieve a desired pressure profile. For example, because substantially all of the pressure drop in the filtrate channel 210 occurs over the length of the ramp, variations in the angle or length of the ramp 216 will result in variations in the pressure profile. In some implementations, more than one ramp 216 could be included within the filtrate channel 210. Other features can also be added to filtrate channel 210 to achieve a desired pressure profile. Several exemplary features are discussed further below.

The pressure in blood flow channel 208 varies linearly along the length of blood flow channel 208, as shown by the line 320. Because the blood flow channel 208 carries blood, it is desirable to avoid sharp increases or decreases in pressure within the blood flow channel 208, in order to promote blood health. The pressure profile in the blood flow channel 208 is determined by its cross-sectional area. As long as the blood flow channel 208 has a uniform cross-sectional area without any additional restrictions, the pressure profile can be maintained as shown in Figure 3, with a relatively high pressure at the upstream portion of the blood flow channel 208 transitioning linearly to a relatively low pressure at the downstream portion of blood flow channel 208.

The channels 208 and 210 are separated by the porous membrane 204, which allows fluid and some particles to be exchanged between the channels 208 and 210. The amount of fluid transferred is proportional to the pressure difference between the channels 208 and 210. Figure 3 shows a graph forward filtration region 326, where the pressure in the blood flow channel 208 carrying blood exceeds the pressure in the in the filtrate channel 210 carrying filtrate. In this region, which corresponds to the channel forward filtration region 226 of Figure 2, the higher pressure in blood flow channel 208 will cause a portion of the fluid in the blood to be transported through the membrane into the filtrate channel 210, along with particles whose diameters are smaller than the diameters of the pores 206. As discussed above, the size of the pores 206 can be selected to allow clearance of particles with a molecular weight of less than about 60 kDa, for example to filter out pathogens, toxins, pollutants, or other undesired substances in the blood while retaining blood cells and platelets in the blood flow channel 208.

Figure 3 also shows a graph back filtration region 328, in which the pressure in filtrate channel 210 carrying filtrate exceeds the pressure in blood flow channel 208 carrying blood. In this region, which corresponds to the channel back filtration region 228 of Figure 2, the higher pressure in filtrate channel 210 causes fluid to be transported from filtrate channel 210, through the membrane 204, and into blood flow channel 208. It is desirable to increase the difference in pressure between the blood channel 208 and the filtrate channel 210 in the graph forward filtration region 326 and in the graph back filtration region 328 of the graph 300, as this difference is proportional to the amount of convective clearance of particles from the blood. The pressure difference is illustrated by the area between the line labeled 318 and line labeled 320 in Figure 3. In some implementations, the device 200 is configured so that the maximum pressure difference in the filtrate channel 210, indicated on the graph 300 by the difference between the maximum and minimum vertical height of line 318, can be maintained within a range of about 200 mmHg to about 2000 mmHg.

As shown in Figures 2 and 3, the addition of the ramp 216 to the filtrate channel 210 increases pressure difference between the blood flow channel 208 and the filtrate channel 210, as indicated in the graph 300 by the graph regions 326 and 328, by maintaining a higher pressure in the graph back filtration region 328 and a lower pressure in the graph forward filtration region 326. Without the ramp 216, the pressure in the filtrate channel 210 would decrease linearly along the length of the filtrate channel 210 from left to right, and the size of both the graph forward filtration region 326 and graph back filtration region 328 would be reduced. Ideally, the volume of blood lost from the blood flow channel 208 in the forward filtration 326 region will be replaced by filtrate in the graph back filtration region 328. For this reason, it is desirable to configure the channels such that the volume of blood lost in forward filtration is equally to the volume of filtrate replaced in back filtration. This is indicated on the graph 300 by the substantially equal areas of the graph forward filtration region 326 and the graph back filtration region 328. It will be understood by one skilled in the art that the sizes of the forward filtration region 328 and the back filtration region 326 shown on the graph 300 do not necessarily correspond to the physical dimensions of the device 200.

The opposite flow directions in the blood flow channel 208 and the filtrate channel 210, as indicated by the arrows 212 and 214, respectively, ensure that undesirable particles filtered from the blood do not return to the blood flow channel 208. Particles are transported from the blood flow channel 208 to the filtrate channel 210 in the forward filtration region, which corresponds to an upstream portion of the blood flow channel 208 (the right-hand side of the Figure 2). Thus, particles are directly carried out of the filtrate channel 210 by the flow of filtrate in the direction of arrow 214. In the back filtration region 326, which corresponds to a downstream portion of the blood flow channel 208 (the left-hand side of Figure 2), fewer if any particles will exist in the filtrate channel 210. The result is that fresh filtrate from the filtrate channel 210 will enter the blood flow channel 208 in the back filtration region 326, and particles that are filtered from the blood flow channel 208 will not reenter the blood flow channel 208.

Figure 2 shows channels 208 and 210 as having rectangular cross-sections. However, other cross-sectional shapes can be used. For example, each blood flow channel 208 and 210 can have a substantially semi-circular cross-section. In other implementations, the channels 208 and 210 may have square or trapezoidal cross sections. In still other implementations, the cross sections of the channels 208 and 210 can be irregular in shape. For example, the channels 208 and 210 may be generally rectangular with rounded or faceted corners, or may contain features (e.g., the ramp 216) that tend to restrict the flow of fluid within the channels. Each blood flow channel 208 and 210 is created by etching, milling, stamping, plating, direct micromachining, or injection molding. In some implementations, the filtrate channel 210 is manufactured such that the ramp 216 is formed by tapering the width of the filtrate channel 210 (e.g., by tapering the width of filtrate channel 210 to form ramp 216 on its bottom wall, as shown in Figure 2). In other implementations, the filtrate channel 210 is manufactured with uniform cross-sectional area along its length, and the ramp 216 is created separately and inserted into the filtrate channel 210.

Although Figure 2 shows the ramp 216 positioned on the bottom wall of the filtrate channel 210, other positions for the ramp 216 are possible. For example, the ramp 216 could be positioned on the side walls of the filtrate channel 210. In some implementations, other pressurizing features may be substituted for, or used in conjunction with, the ramp 216. For example, a pressurizing feature may include bypass channels that carry fluid out of the filtrate channel 210, through the pressurizing feature, and back into the filtrate channel 210. Various alternative pressurizing features are described below.

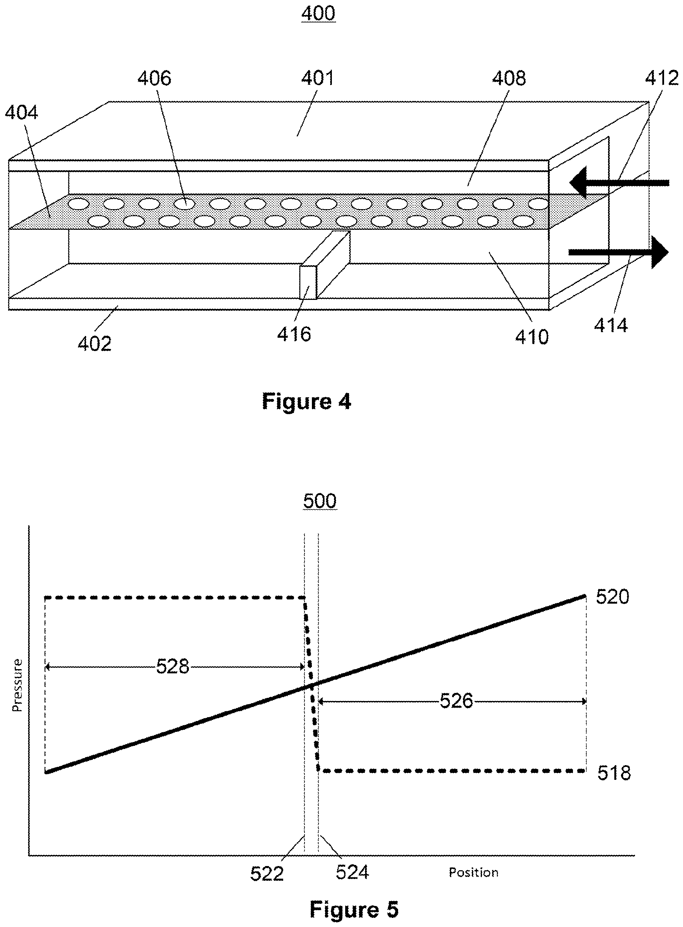

Figure 4 is a depiction of an alternative channel bilayer configuration 400 suitable for use in the microfluidic convective clearance device of Figure 1A. The blood substrate layer 401 has a blood flow channel 408 and the filtrate substrate layer 402 has a filtrate channel 410. The channels 408 and 410 are separated by the membrane 404, and fluid is transported through the channels 408 and 410 in the direction shown by the arrows 412 and 414. The filtrate channel 410 also includes a partial wall 416 configured to restrict the flow of fluid through the filtrate channel 410.

As described above in connection with Figures 2 and 3, a non-linear pressure profile in filtrate channel 410 can help to increase the amount of convective clearance of particles in blood flow channel 408. The partial wall 416 can be used to create such a non-linear pressure profile. The partial wall 416 extends across the entire width of filtrate channel 210 and extends upwards toward the membrane 404. Because the partial wall 416 is impermeable to the filtrate fluid, it decreases the cross-sectional area of filtrate channel 410 through which fluid flows. The restriction created by the partial wall 416 therefore results in a high pressure at an upstream portion of the filtrate channel 410 and a low pressure at a downstream portion of the filtrate channel 410. Substantially all of the pressure drop within the filtrate channel 410 occurs across the partial wall 416.

The dimensions of the partial wall 416 can be selected to achieve a desired pressure profile. For example, the restriction to fluid flow imposed by the partial wall 416 increases as the height of the partial wall increases. Therefore, a higher pressure at the upstream portion of the filtrate channel 410 can be created by increasing the height of the partial wall 410. Similarly, the a lower pressure at the upstream portion of the filtrate channel 410 can be created by reducing the height of the partial wall 416.

Because the pressure drop in the filtrate channel 410 occurs as fluid moves across the top of the partial wall 416, the length of the partial wall 416 (in the direction of the length of the filtrate channel 410) can also impact the pressure profile. For example, an increased length of the partial wall 416 allows the pressure to decrease more gradually, because the pressure drop occurs over a longer portion of the filtrate channel 410. A shorter length for the partial wall 416 causes a the pressure to drop more rapidly. For an arbitrarily short length of the partial wall 416, the pressure profile in the filtrate channel 410 is substantially a step function, in which the step occurs at the partial wall 416. In some implementations, the pressure profile can be adjusted by adjusting the height of the partial wall 416. For example, the partial wall 416 can be adjustably inserted through the bottom of the filtrate channel 410. Extending the partial wall 416 further into the filtrate channel 410 can effectively increase the height of the partial wall 416.

Figure 5 is a graph 500 of the fluid pressure along the channels of the bilayer configuration depicted in Figure 4. The line 518 represents the fluid pressure in the filtrate channel 410 and the line 520 represents the fluid pressure in the blood flow channel 408. The partial wall 416 lies along the bottom of the filtrate channel 410 between the points labeled 522 and 524 on the position axis of Figure 5. As shown, the partial wall 416 creates a non-linear pressure profile along the length of the filtrate channel 410. To the left of point 522, the fluid pressure in filtrate channel 410 is high and relatively constant, due to the flow restriction imposed by the partial wall 416. As fluid flows over the partial wall 416, the pressure decreases until it reaches a minimum level at the end of the partial wall 416, where the filtrate channel 210 has a relatively wide cross section. In this implementation, the relatively narrow width of the partial wall 416 creates a sharp pressure drop in the filtrate channel 410. As a result, the pressure profile in the filtrate channel 410 is substantially a step function, as indicated by the line 518.

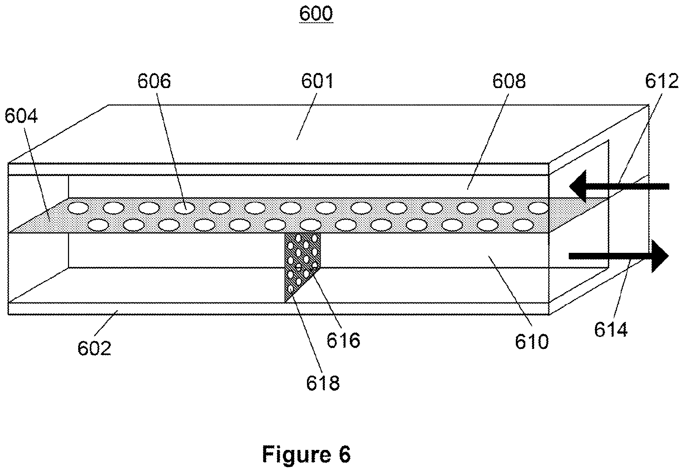

Figure 6 is a depiction of an alternative channel bilayer configuration 600 suitable for use in the microfluidic convective clearance device of Figure 1A. The blood substrate layer 601 has a blood flow channel 608 and the filtrate substrate layer 602 has filtrate channel 610. Channels 608 and 610 are separated by the membrane 604, and fluid is transported through the channels 608 and 610 in the direction shown by the arrows 612 and 614. The filtrate channel 610 also includes a second membrane 616 with pores 618 configured to restrict the flow of fluid through the filtrate channel 610.

The membrane 616 can be selected to achieve a desired pressure profile along the length of the filtrate channel 610. For example, the size of the pores 618 can be decreased to further restrict fluid flow, resulting in an increased pressure upstream from the membrane 616, or can be decreased to decrease the upstream pressure. The number of the pores 618 and the spacing of the pores 618 can also be varied.

The relative size of the pores 606 of the membrane 604 and the pores 618 or the second membrane 616 are not necessarily drawn to scale in Figure 6. In some implementations, the pores 606 may have a different size than the pores 618. Particles are transferred from the blood flow channel 608 into the filtrate channel 610 through the membrane 606 only in the forward filtration region, which occurs at the downstream portion of the filtrate channel 610 (the right-hand side of filtrate channel 610, as shown in Figure 6). Therefore, the pores 618 can be smaller than the pores 606, because particles that travel through the pores 606 will not also have to travel through the pores 618.

In some implementations, the membrane 618 may be replaced by another feature that serves a similar purpose. For example, a porous plug or a gel may be inserted into the filtrate channel 610 to restrict the flow of filtrate through the filtrate channel 610, thereby yielding a high pressure at an upstream portion of the filtrate channel 610 and a low pressure at a downstream portion of the channel 610.

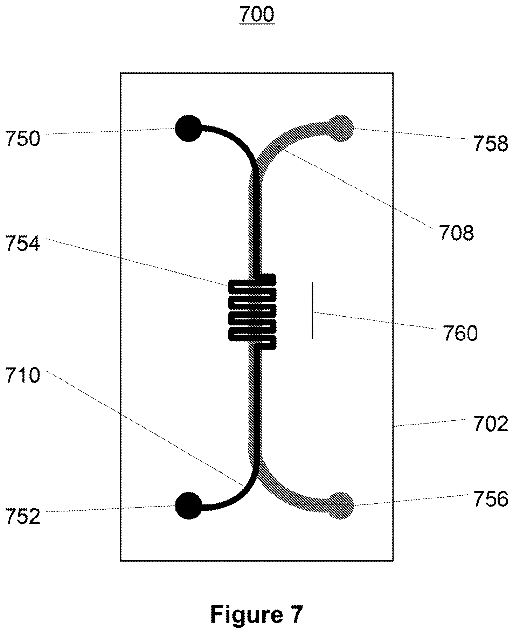

Figure 7 is a depiction of an alternative channel bilayer configuration 700 suitable for use in the microfluidic convective clearance device of Figure 1A. A filtrate substrate layer 702 has a filtrate channel 710. The filtrate channel 710 includes an inlet 750, an outlet 752, and a circuitous region 754. Also shown in Figure 7 is a blood flow channel 708, which is located beneath the filtrate channel 710. The blood flow channel 708 includes an inlet 756 and an outlet 758. The blood flow channel 708 can be contained within a blood substrate layer beneath the filtrate substrate layer 702. The channels 708 and 710 are separated by a permeable membrane, which is not shown in Figure 7.

The inlet 750 of the filtrate channel 710 is opposite the inlet 756 of the blood flow channel 708 to allow the fluid in channels 708 and 710 to flow in opposite directions. The distance between the inlet 750 and the outlet 752 of the filtrate channel 710 is approximately equal to distance between the inlet 756 and the outlet 758 of the blood flow channel 708. However, the total length of the filtrate channel 710 is substantially longer than the total length of the blood flow channel 208 due to the circuitous region 754. In addition, the length 760 of the circuitous region 754 is substantially shorter than the distance between the inlet 750 and the outlet 752 of the filtrate channel 710. This results in a non-linear pressure profile along the length of filtrate channel 710, including a large pressure drop across the circuitous region 754, yielding a high pressure in the filtrate channel 710 upstream from the circuitous region 754 and a low pressure downstream from the circuitous region 754. Characteristics of the circuitous path 754, such as the length 760 or the number of turns, can be selected to achieve a desired pressure profile in the filtrate channel 710. The non-linear pressure profile increases the amount of convective clearance of particles from blood flow channel 708 to filtrate channel 710 through the permeable membrane.

The pressurizing features described above in connection with Figures 2, 4, 6, and 7, such as the ramp 216, the partial wall 416, the membrane 616, or the circuitous region 754, may be combined into a single implementation. Other pressurizing features may also be used. For example, any feature that extracts work from the fluid flowing through a channel, such as a reverse pump or a turbine, could also be used as a pressurizing feature.

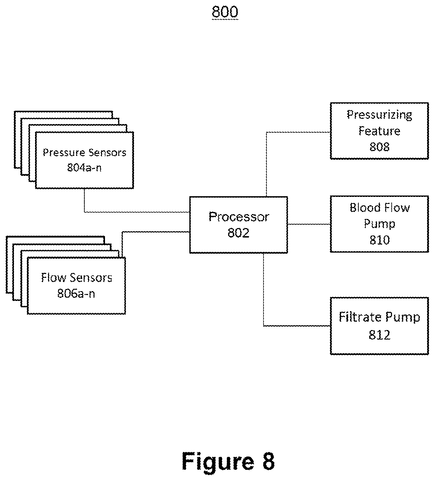

Figure 8 is a depiction of a system 800 for cleansing a fluid incorporating the microfluidic convective clearance device 100 of Figure 1A. The system 800 includes a processor 802 which receives input from a plurality of fluid pressure sensors 804 and a plurality of flow sensors 806. The processor 802 is also in communication with pressurizing feature 808, blood flow pump 810, and filtrate pump 812. In some implementations, the pressure sensors 804 and the flow sensors 806 can be located inside the channels of the device. The processor 804 can thus monitor pressure and flow, and can respond by adjusting the pressurizing feature 808 or the output rate of the pumps 810 and 812. For example, the processor can adjust the pressurizing feature 808 or the pumps 810 and 812 to maintain pressures within the blood flow channel that are safe for preserving blood health.

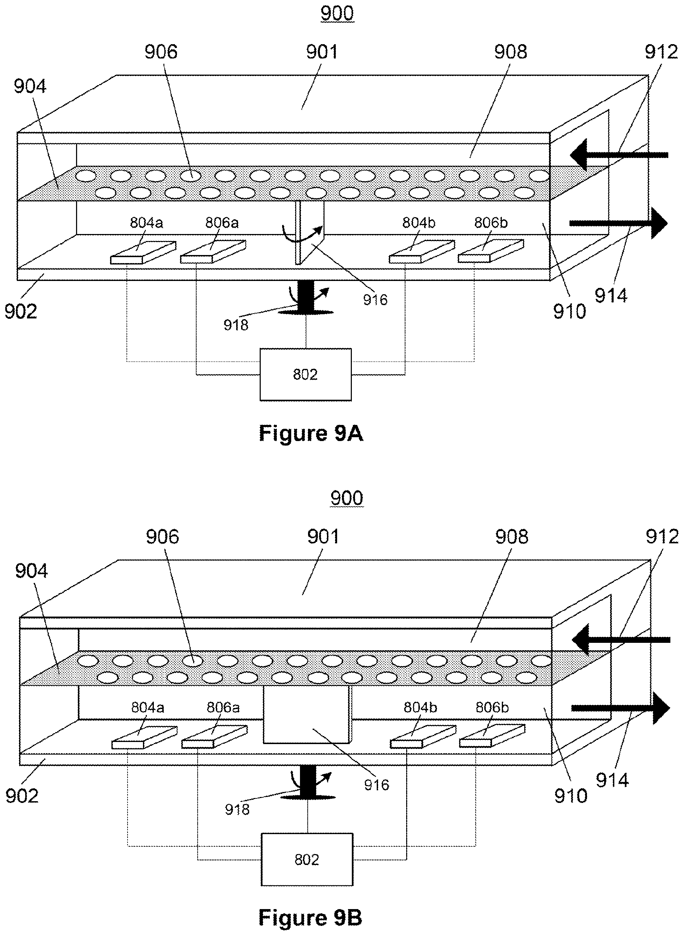

Figure 9A is a depiction of an alternative channel bilayer configuration 900 suitable for use in the fluid cleansing system of Figure 8, in a first state. Like the channel bilayer configurations 200, 400, 600, and 700 of Figures 2, 4, 6, and 7, respectively, the channel bilayer configuration 900 includes a blood substrate layer 901 and a filtrate substrate layer 902. The blood substrate layer 901 has a blood flow channel 908 and the filtrate substrate layer 902 has a filtrate channel 910. Channels 908 and 910 are separated by a membrane 904, and fluid is transported through the channels 908 and 910 in the directions shown by the arrows 912 and 914. The filtrate channel 910 also includes an upstream pressure sensor 804a, a downstream pressure sensor 804b, an upstream flow sensor 806a, and a downstream flow sensor 806b, which are in communication with a processor 802, as described above in connection with Figure 8. The processor 802 is also in communication with a valve 916 inside the filtrate channel 910, which is configured to restrict the flow of fluid through the filtrate channel 910. In this example, the valve 916 corresponds to the pressurizing feature 808 of Figure 8.

The valve 916 is shown in Figure 9A as a butterfly valve connected to a rotary actuator 918, but any type of throttling device, including other types of valves, could be used as well. The valve 916 has the same width and height as the filtrate channel 910. In Figure 9A, the valve 916 is shown in a partially closed position, which substantially restricts the flow of fluid within the filtrate channel 910. Rotation of the rotary actuator 918 causes the valve 916 to rotate about its center axis, allowing the valve 916 to be opened and closed.

Figure 9B is a depiction of the alternative channel bilayer configuration 900 suitable for use in the fluid cleansing system of Figure 8, in a second state (i.e., with the valve 916 in a fully opened position). The relatively small thickness of the valve 916 presents a minimal restriction to fluid flow in the open position. Although not shown in the figures, other positions of the valve are possible (i.e., the valve may be rotated at different angles).

As discussed above in connection with Figures 2, 4, 6, and 7, a restriction within the filtrate channel 910 can create a non-linear pressure profile along the length of the filtrate channel 910. The non-linear pressure profile can result in increased convective clearance of particles from the blood flow channel 908 through the membrane 904. For example, positioning the valve 916 such that it is nearly fully closed will allow only a small amount of fluid to flow past the valve, resulting in a high pressure upstream from the valve 916, a low pressure downstream from the valve 916, and rapid pressure drop across the valve 916. Opening the valve 916 from this position will reduce the fluid restriction imposed by the valve 916. When the valve 916 is fully opened, the pressure profile in the filtrate channel 910 will be substantially a linear decrease from the upstream portion to the downstream portion.

The filtrate channel 910 also includes pressure sensors 804a and 804b for determining fluid pressure and flow sensors 806a and 806b for determining fluid flow rates. In some implementations, these sensors may have a low profile, or may otherwise be designed to interfere minimally with the fluid flow in the filtrate channel 910. Because the pressure and flow rate in the filtrate channel 910 can be considerably different on either side of the flow restriction (i.e., the valve 916), two pressure sensors 804a and 804b and two flow sensors 806a and 806b are included, with one of each type of sensor installed at an upstream portion of the filtrate channel 910 and another of each type of sensor installed at a downstream portion.

The pressure sensors 804a and 804b and the flow sensors 806a and 806b can be communicatively coupled to a processor 802. The processor 802 can also be communicatively coupled to the rotary actuator 918. This configuration allows the position of the valve 916 to be varied over time in response to measured variations in pressure and flow rate within the filtrate channel 910. For example, if the pressure difference between the upstream portion and the downstream portion of the filtrate channel 910 exceeds a maximum threshold, the processor 802 can respond by rotating the rotary actuator 918 to put the valve 916 in a more open position, thereby reducing the pressure. Similarly, the processor 802 can determine that the flow rate downstream from the valve 916 is above a maximum threshold, based on the measurement from downstream flow detector 806b. The processor 802 could respond to this condition by controlling rotary actuator 918 to put the valve 916 in a more closed position, in order to decrease the flow rate. The processor 802 could also communicate to a fluid introduction device, such as a pump, to increase or decrease the flow rate at which filtrate is introduced into the filtrate channel 910. In other implementations, the rotary actuator 918 or a fluid introduction device can be manually operated (e.g., by a physician or a patient).

In other implementations, the filtrate substrate layer 902 can be made from a flexible material, and a non-linear pressure profile can be created in the filtrate channel 910 by causing a portion of the material of the filtrate substrate layer 902 to deform inwards toward the membrane 904. For example, deformation of a portion of the filtrate substrate layer 902 can reduce the cross-sectional area of a portion of the filtrate channel 910. The reduced cross-sectional area can restrict fluid flow in the filtrate channel 902, resulting in a high pressure upstream from the deformed portion of the filtrate channel 902 and a low pressure downstream from the deformed portion of the filtrate channel 902. In one example, the deformation of the filtrate substrate layer 902 can be controlled by the processor 802, and can be adjusted in response to measurements made by the pressure sensors 804a and 804b and the flow sensors 806a and 806b.

In some implementations, the blood flow channel 908 can also include pressure and flow sensors, which can be communicatively coupled to the processor 902. Pressure and flow sensors can be used to ensure that the fluid characteristics in the blood flow channel 908 are maintained within ranges that preserve blood health. For example, if a pressure sensor detects an unacceptably high pressure in the blood flow channel 908, the processor 802 could respond by turning the rotary actuator 918 to put the valve 916 in a more open position, reducing the pressure upstream from the valve 916 in the filtrate channel 910. This decreases the amount of fluid transferred from the filtrate channel 910 to the blood flow channel 908, thereby decreasing the pressure in the blood flow channel 908. The pressure and flow characteristics could also be adjusted to maintain the wall shear rate in an acceptable range for blood. In some implementations, the pressure and flow characteristics can be adjusted to achieve a desired level of convective clearance of particles from the blood flow channel 908. For example, increasing the pressure in the filtrate channel 910 can result in an increased level of convective clearance, and decreasing the pressure in the filtrate channel 910 can result in a decrease in the level of convective clearance, as discussed above in connection with Figures 2 and 3. The processor 802 can therefore control the rotary actuator 918 to put the valve 916 in a more closed position, thereby increasing the pressure in the filtrate channel 910 and increasing the amount of convective clearance. Alternatively, the processor 802 can control the rotary actuator 918 to put the valve 916 in a more open position, which reduces the pressure in the filtrate channel 910 and therefore decreases the amount of convective clearance.

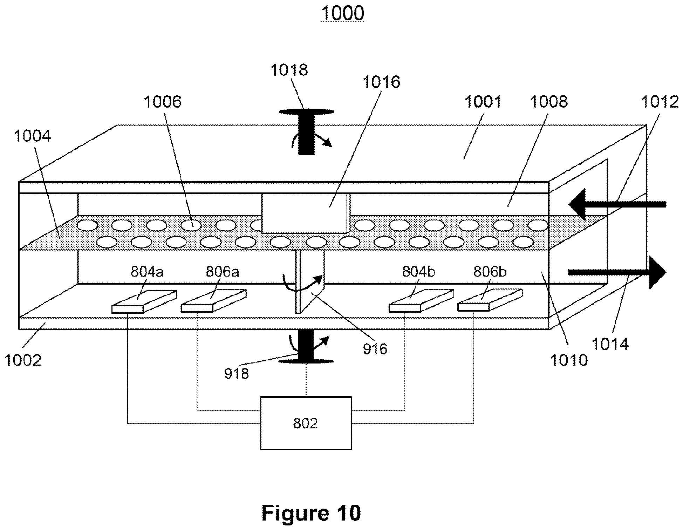

Figure 10 is a depiction of an alternative channel bilayer configuration 1000 suitable for use in the fluid cleansing system of Figure 1A, according to an illustrative implementation. Like the channel bilayer configuration 900 of Figure 9, the channel bilayer configuration 1000 includes a blood substrate layer 1001 and a filtrate substrate layer 1002. The blood substrate layer 1001 has a blood flow channel 1008 and the filtrate substrate layer 1002 has a filtrate channel 1010. Channels 1008 and 1010 are separated by a membrane 1004, and fluid is transported through the channels 1008 and 1010 in the directions shown by the arrows 1012 and 1014. The filtrate channel 1010 also includes an upstream pressure sensor 804a, a downstream pressure sensor 804b, an upstream flow sensor 806a, and a downstream flow sensor 806b, which are in communication with a processor 802, as described above in connection with Figure 8. The processor 802 is also in communication with a valve 916 inside the filtrate channel 1010, which is configured to restrict the flow of fluid through the filtrate channel 1010, as describe above in connection with Figures 9A and 9B.

In contrast to the bilayer configuration 900 of Figure 9, the bilayer configuration 1000 shown in figure 10 includes an additional pressurizing feature in the blood flow channel 1008. Specifically, in Figure 10, a the pressurizing feature in the blood flow channel 1008 takes the form of a second valve 1016 connected to a second rotary actuator 1018. Like the valve 916, the second valve 1016 has the same width and height as the blood channel 1008. In Figure 10, the second valve 1016 is shown in a fully open position. Rotation of the rotary actuator 1018 causes the second valve 1016 to rotate about its center axis, allowing the second valve 1016 to be opened and closed. In some implementations, the second valve 1016 can be controlled independently from the valve 916. For example, as shown in Figure 10, the second valve 1016 can be fully opened while the valve 916 is partially closed. The second valve 1016 can be controlled by manual actuation of the rotary actuator 1018, or can be controlled electronically by the processor 802. In some implementations, sensor similar to the pressure sensors 804a and 804b and the flow sensors 806a and 806b can be located in the blood channel 1008, and the second valve 1016 can be actuated in response to the measurements taken by the sensors. Although a butterfly valve 1016 is shown in Figure 10, any other type of channel restriction can be located within the blood channel 1008, including any of the channel restrictions discussed above. In some implementations, more than one channel restriction can be located in the blood channel 1008.

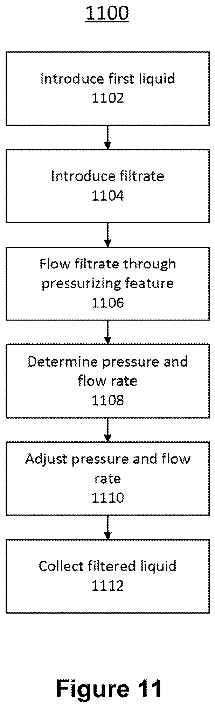

Figure 11 is a flow diagram of a method 1100 for filtering a first liquid containing an analyte to provide a filtered liquid containing less analyte than the first liquid. The method 1100 includes introducing a first liquid (step 1102), introducing filtrate (step 1104), flowing filtrate through a pressurizing feature (step 1106), determining pressure and flow rates (step 1108), adjusting pressure and flow rates (step 1110), and collecting filtered liquid (step 1112).

The method 1100 includes the step of introducing a first liquid into network of channels have one or more first channels (step 1102). In some implementations, the fluid is blood that has been extracted from a patient for filtration. The analyte in the blood can be urea, uric acid, or creatinine. The first channels can have a height in the range of about 50 microns to about 500 microns, a width in the range of about 50 microns to about 900 microns, and a length in the range of about 3 centimeters to about 20 centimeters. If blood is to be introduced into the first channel, the first channel can include an anticoagulant coating on its inner walls.

The method 1100 includes the step of introducing filtrate into an inlet of at least one second channel (step 1104). The second channel is complementary to one or more of the first channels, and the inlet of the second channel is at the opposite end of the channels with respect to the inlet of the first channel, such that the filtrate introduced into the second channel flows in a direction opposed to the direction of the first liquid in the first channel. The second channel is separated from the one or more complementary first channels by a permeable membrane, which allows some of the analyte to be transferred from the first channel to the second channel.

The method 1100 includes the step of flowing the filtrate through a pressurizing feature in the at Second Channel (step 1106). The pressurizing feature of the second channel is configured to yield a high pressure in a portion of the second channel upstream from the pressurizing feature, a low pressure in a portion of the second channel downstream from the pressurizing feature, and a non-linear pressure profile along the length of the second channel. For example, the pressurizing feature can be an obstruction inserted into the second channel to restrict the flow of fluid, such as the ramp, partial wall, membrane, or valve discussed above. The pressurizing feature could also be a circuitous path followed by the second channel over a portion of its length, as described in connection with Figure 7. The second channel may contain more than one pressurizing feature. In some implementations, the pressurizing feature is configured to yield a pressure profile in the second channel that is substantially a step function. The non-linear pressure profile results in increased convective clearance of analyte from the first channel to the second channel through the permeable membrane.

The method 1100 includes the step of determining the pressure and flow rates in the first and second channels (step 1108). This can be achieved by placing pressure and flow sensors inside one or both of the first channel and the second channel. Because the pressure profile in the second channel is non-linear, the second channel can include pressure and flow sensors on either side of the pressurizing feature. In some implementations, the pressure and flow sensors can communicate with an electronic processor.

The method 1100 includes the step of adjusting the pressure or flow rates of the first or second channels (step 1110). The pressure and flow rates can be adjusted in response to the pressure and flow rates determined in step 1108. For example, the pressure in the second channel can be adjusted by changing the characteristics of pressurizing feature of the second channel, such as by opening or closing a valve in the second channel. The pressure or flow rate can also be adjusted by limiting the amount of fluid introduced into the first channel or the second channel, for example by controlling a fluid introduction device for each channel. As discussed above, the pressure and flow sensors can be communicatively coupled to an electronic processor. The processor can in turn be coupled to the pressurizing feature or the fluid introduction devices to control the pressure and flow rate in the first and second channels. Alternatively, a human operator could manually control the pressurizing feature or fluid introduction devices in response to the pressure and flow rates measured by the pressure and flow sensors in step 1108. In some implementations, the pressure and flow rates can be adjusted to preserve the health of blood flowing in the first channel.

The method 1100 also includes the step of collecting the filtered liquid from an outlet of the first channel (step 1112). As discussed above, the complementary first and second channel are configured such that fluid flows in opposite directions through the channels. The outlet of the first channel where the filtered liquid is collected is therefore situated near the inlet of the second channel. The pressurizing feature in the second channel yields a lower pressure downstream from the pressurizing feature in the second channel than the pressure in the corresponding section of the first channel. This allows analyte to be transported from the first channel to the second channel through the permeable membrane. The pressurizing feature in the second channel also yields a higher pressure upstream from the pressurizing feature in the second channel than the pressure in the corresponding section of the first channel, which causes some of the filtrate to be transported from the second channel to the first channel through the permeable membrane. The result is that analyte and fluid from the first channel are removed, filtrate replaces the lost fluid in the first channel, and the liquid collected at the outlet of the first channel therefore contains a lower concentration of analyte than the liquid at the inlet of the first channel.

Having now described some illustrative implementations, it is apparent that the foregoing is illustrative and not limiting, having been presented by way of example. In particular, although many of the examples presented herein involve specific combinations of method acts or system elements, those acts and those elements may be combined in other ways to accomplish the same objectives. Acts, elements and features discussed only in connection with one implementation are not intended to be excluded from a similar role in other implementations.

The systems and methods described herein may be embodied in other specific forms without departing from the characteristics thereof. The foregoing implementations are illustrative rather than limiting of the described systems and methods. Scope of the systems and methods described herein is thus indicated by the appended claims, rather than the foregoing description, and changes that come within the meaning and range of equivalency of the claims are embraced therein.

## Claims

1. A microfluidic device (100), comprising:
a network of channels having one or more First Channels (208; 408; 608; 708), each First Channel having a height in the range of 50 microns to 500 microns, a width in the range of 50 microns to 900 microns, and a length in the range of 3 centimeters to 20 centimeters;
at least one Second Channel (210; 410; 610; 710) complementary to one or more of the First Channels, the at least one Second Channel comprising at least one pressurizing feature (216; 416; 616; 754; 916) configured to yield a high pressure in a portion (228) of the at least one Second Channel upstream from the pressurizing feature and a low pressure in a portion (226) of the at least one Second Channel downstream from the pressurizing feature; and
a filtration membrane (204; 404; 604) separating the one or more First Channels from the at least one Second Channel, wherein the upstream portion of the at least one Second Channel is located opposite a downstream portion of the one or more complementary First Channels, such that when fluid is flowed through the First and Second Channels, a non-linear pressure profile (318; 518) exists along the length of the at least one Second Channel and a pressure gradient exists across the membrane separating the one or more First Channels from the at least one complimentary Second Channel.

2. The microfluidic device of claim 1, wherein the one or more First Channels (1008) comprise at least one second pressurizing feature (1016) configured to yield a high pressure in a portion of the one or more First Channels upstream from the pressurizing feature and a low pressure in a portion of the one or more First Channels downstream from the pressurizing feature.

3. The microfluidic device of claim 1, wherein the non-linear pressure profile along the length of the at least one Second Channel is substantially a step function.

4. The micro fluidic device of claim 1, wherein the pressurizing feature comprises one of:
• a partial wall (416) configured to restrict fluid flow in the at least one Second Channel, yielding the high pressure at an upstream portion of the at least one Second Channel and the low pressure at a downstream portion of the at least one Second Channel;
• a section (216) of the at least one Second Channel that tapers to reduce the cross-sectional area of a portion of the channel, yielding the high pressure at an upstream portion of the at least one Second Channel and the low pressure at a downstream portion of the at least one Second Channel;
• a section (754) of the at least one Second Channel configured to direct fluid along a circuitous path, yielding the high pressure at an upstream portion of the at least one Second Channel and the low pressure at a downstream portion of the at least one Second Channel;
• at least one of a porous plug (616), a second membrane, and a gel inserted into the at least one Second Channel, yielding the high pressure at an upstream portion of the at least one Second Channel and the low pressure at a downstream portion of the at least one Second Channel;;
• a device that extracts work from a flow of fluid in the at least one Second Channel, thereby yielding the high pressure at an upstream portion of the at least one Second Channel and the low pressure at a downstream portion of the at least one Second Channel, wherein the device is one of a reverse pump and a turbine located inside the at least one Second Channel; or
• a throttling device (916) coupled to the at least one Second Channel and configured to control a flow of fluid in the at least one Second Channel, thereby yielding the high pressure at an upstream portion of the at least one Second Channel and the low pressure at a downstream portion of the at least one Second Channel, wherein the throttling device includes a valve.

5. The microfluidic device of claim 1, wherein the at least one Second Channel is formed from a flexible material, and wherein the pressurizing feature comprises a portion of the at least one Second Channel that can be compressed to reduce its cross-sectional area, thereby yielding the high pressure at an upstream portion of the at least one Second Channel and the low pressure at a downstream portion of the at least one Second Channel.

6. The micro fluidic device of claim 1, wherein the pressurizing feature is configurable to provide a desired pressure profile in the at least one Second Channel.

7. The micro fluidic device of claim 1, further comprising at least one of a flow rate sensor (806a, 806b) and a pressure sensor (804a, 804b) for determining at least one of a flow rate and a fluid pressure in the one or more First Channels and the at least one Second Channel.

8. The micro fluidic device of claim 7, further comprising a processor (802) configured to control the pressurizing feature responsive to the determined flow rate or fluid pressure.

9. The microfluidic device of claim 1, wherein the device is configured to maintain a maximum pressure difference in the at least one Second Channel in a range of 200 mmHg to 2000 mmHg.

10. The micro fluidic device of claim 1, wherein the membrane has a thickness in the range of 5 µm to 300 µm.

11. The micro fluidic device of claim 1, wherein the pore size of the membrane is selected to allow clearance of particles with a molecular weight of no more than about 60 kDa.

12. The microfluidic device of claim 1, further comprising an anticoagulant coating on the inner surfaces of the one or more First Channels.

13. The microfluidic device of claim 1, wherein the one or more First Channels are configured to maintain wall shear rates in the range of 200 inverse seconds to 2000 inverse seconds when blood is transported through the one or more First Channels.

14. The microfluidic device of claim 1, wherein the one or more First Channels and the at least one Second Channel are configured for flowing fluid in opposite directions.

15. A method for filtering a first liquid containing an analyte to provide a filtered liquid containing less analyte than the first liquid, the method comprising the steps of:
introducing (1102) the first liquid into an inlet of a network of channels having one or more First Channels, each First Channel having a height in the range of 50 microns to 500 microns, a width in the range of 50 microns to 900 microns, and a length in the range of 3 centimeters to 20 centimeters;
introducing (1104) filtrate into an inlet of at least one Second Channel complementary to the one or more First Channels, a filtration membrane (204; 404; 604) separating the one or more First Channels from the at least one Second Channel;
flowing (1106) the filtrate through at least one pressurizing feature in the at least one Second Channel, wherein the at least one pressurizing feature is configured to yield a high pressure in a portion of the at least one Second Channel upstream from the pressurizing feature, a low pressure in a portion of the at least one Second Channel downstream from the pressurizing feature, and a non-linear pressure profile along the length of the at least one Second Channel, such that at least some of the analyte of the first liquid is transported from the one or more First Channels through the filtration membrane and into the at least one Second Channel; and
collecting (1112) the filtered liquid from an outlet of one or more of the First Channels, wherein the outlet of the one or more First Channels is located opposite the inlet of the at least one corresponding Second Channel, and an inlet of the one or more First Channels is located opposite an outlet of the at least one complimentary Second Channel.

## Patentansprüche

1. Mikrofluidische Vorrichtung (100), umfassend:
ein Netzwerk von Kanälen mit einem oder mehreren ersten Kanälen (208; 408; 608; 708), wobei jeder erste Kanal eine Höhe im Bereich von 50 Mikrometer bis 500 Mikrometer, eine Breite im Bereich von 50 Mikrometer bis 900 Mikrometer und eine Länge im Bereich von 3 Zentimeter bis 20 Zentimeter aufweist;
mindestens einen zweiten Kanal (210; 410; 610; 710), der zu einem oder mehreren der ersten Kanäle komplementär ist, wobei der mindestens eine zweite Kanal mindestens ein Druckmerkmal (216; 416; 616; 754; 916) umfasst, das konfiguriert ist, um in einem dem Druckmerkmal vorgelagerten Abschnitt (228) des mindestens einen zweiten Kanals einen Hochdruck und in einem dem Druckmerkmal nachgelagerten Abschnitt (226) des mindestens einen zweiten Kanals einen Niederdruck zu ergeben; und
eine Filtrationsmembran (204; 404; 604), die den einen oder die mehreren ersten Kanäle von dem mindestens einen zweiten Kanal trennt, wobei der vorgelagerte Abschnitt des mindestens einen zweiten Kanals einem nachgelagerten Abschnitt des einen oder der mehreren komplementären ersten Kanäle entgegengesetzt angeordnet ist, sodass, wenn Fluid durch die ersten und zweiten Kanäle fließt, entlang der Länge des mindestens einen zweiten Kanals ein nichtlineares Druckprofil (318; 518) besteht und über die Membran, welche den einen oder die mehreren ersten Kanäle von dem mindestens einen komplementären zweiten Kanal trennt, ein Druckgradient besteht.

2. Mikrofluidische Vorrichtung nach Anspruch 1, wobei der eine oder die mehreren ersten Kanäle (1008) mindestens ein zweites Druckmerkmal (1016) umfassen, das konfiguriert ist, um in einem dem Druckmerkmal vorgelagerten Abschnitt des einen oder der mehreren ersten Kanäle einen Hochdruck und in einem dem Druckmerkmal nachgelagerten Abschnitt des einen oder der mehreren ersten Kanäle einen Niederdruck zu ergeben.

3. Mikrofluidische Vorrichtung nach Anspruch 1, wobei das nichtlineare Druckprofil entlang der Länge des mindestens einen zweiten Kanals im Wesentlichen eine Treppenfunktion ist.

4. Mikrofluidische Vorrichtung nach Anspruch 1, wobei das Druckmerkmal eines von Folgendem umfasst:
• eine Teilwand (416), die konfiguriert ist, um Fluidfluss in dem mindestens einen zweiten Kanal zu beschränken, was den Hochdruck bei einem vorgelagerten Abschnitt des mindestens einen zweiten Kanals und den Niederdruck bei einem nachgelagerten Abschnitt des mindestens einen zweiten Kanals ergibt;
• ein Teilstück (216) des mindestens einen zweiten Kanals, das konisch zuläuft, um die Querschnittsfläche eines Abschnitts des Kanals zu reduzieren, was den Hochdruck bei einem vorgelagerten Abschnitt des mindestens einen zweiten Kanals und den Niederdruck bei einem nachgelagerten Abschnitt des mindestens einen zweiten Kanals ergibt;
• ein Teilstück (754) des mindestens einen zweiten Kanals, das konfiguriert ist, um Fluid entlang einem Schlängelweg zu leiten, was den Hochdruck bei einem vorgelagerten Abschnitt des mindestens einen zweiten Kanals und den Niederdruck bei einem nachgelagerten Abschnitt des mindestens einen zweiten Kanals ergibt;
• mindestens eines von einem porösen Pfropfen (616), einer zweiten Membran und einem Gel, eingeführt in den mindestens einen zweiten Kanal, was den Hochdruck bei einem vorgelagerten Abschnitt des mindestens einen zweiten Kanals und den Niederdruck bei einem nachgelagerten Abschnitt des mindestens einen zweiten Kanals ergibt;
• eine Vorrichtung, die aus einem Fluidfluss in dem mindestens einen zweiten Kanal Arbeit extrahiert, wodurch sich der Hochdruck bei einem vorgelagerten Abschnitt des mindestens einen zweiten Kanals und der Niederdruck bei einem nachgelagerten Abschnitt des mindestens einen zweiten Kanals ergibt, wobei die Vorrichtung eines von einer Umkehrpumpe und einer Turbine, angeordnet innerhalb des mindestens einen zweiten Kanals, ist; oder
• eine Drosselvorrichtung (916), die an den mindestens einen zweiten Kanal gekoppelt ist und konfiguriert ist, um einen Fluidfluss in dem mindestens einen zweiten Kanal zu steuern, wodurch sich der Hochdruck bei einem vorgelagerten Abschnitt des mindestens einen zweiten Kanals und der Niederdruck bei einem nachgelagerten Abschnitt des mindestens einen zweiten Kanals ergibt, wobei die Drosselvorrichtung ein Ventil beinhaltet.

5. Mikrofluidische Vorrichtung nach Anspruch 1, wobei der mindestens eine zweite Kanal aus einem flexiblen Material gebildet ist und wobei das Druckmerkmal einen Abschnitt des mindestens einen zweiten Kanals umfasst, der komprimiert werden kann, um seine Querschnittsfläche zu reduzieren, wodurch sich der Hochdruck bei einem vorgelagerten Abschnitt des mindestens einen zweiten Kanals und der Niederdruck bei einem nachgelagerten Abschnitt des mindestens einen zweiten Kanals ergibt.

6. Mikrofluidische Vorrichtung nach Anspruch 1, wobei das Druckmerkmal konfigurierbar ist, um in dem mindestens einen zweiten Kanal ein gewünschtes Druckprofil bereitzustellen.

7. Mikrofluidische Vorrichtung nach Anspruch 1, ferner umfassend mindestens eines von einem Fließgeschwindigkeitssensor (806a, 806b) und einem Drucksensor (804a, 804b), um mindestens eines von einer Fließgeschwindigkeit und einem Fluiddruck in dem einen oder den mehreren ersten Kanälen und dem mindestens einen zweiten Kanal zu bestimmen.

8. Mikrofluidische Vorrichtung nach Anspruch 7, ferner umfassend einen Prozessor (802), der konfiguriert ist, um als Reaktion auf die bestimmte Fließgeschwindigkeit oder den bestimmten Fluiddruck das Druckmerkmal zu steuern.

9. Mikrofluidische Vorrichtung nach Anspruch 1, wobei die Vorrichtung konfiguriert ist, um einen Maximaldruckunterschied in dem mindestens einen zweiten Kanal in einem Bereich von 200 mmHg bis 2000 mmHg aufrechtzuerhalten.

10. Mikrofluidische Vorrichtung nach Anspruch 1, wobei die Membran eine Dicke im Bereich von 5 µm bis 300 µm aufweist.

11. Mikrofluidische Vorrichtung nach Anspruch 1, wobei die Porengröße der Membran ausgewählt ist, um eine Clearance von Partikeln mit einem Molekulargewicht von nicht mehr als ungefähr 60 kDa zu ermöglichen.

12. Mikrofluidische Vorrichtung nach Anspruch 1, ferner umfassend eine gerinnungshemmende Beschichtung auf den inneren Oberflächen des einen oder der mehreren ersten Kanäle.

13. Mikrofluidische Vorrichtung nach Anspruch 1, wobei der eine oder die mehreren ersten Kanäle konfiguriert sind, um Wandschergeschwindigkeiten im Bereich von 200 reziproken Sekunden bis 2000 reziproken Sekunden aufrechtzuerhalten, wenn Blut durch den einen oder die mehreren ersten Kanäle transportiert wird.

14. Mikrofluidische Vorrichtung nach Anspruch 1, wobei der eine oder die mehreren ersten Kanäle und der mindestens eine zweite Kanal konfiguriert sind, um Fluid in entgegengesetzte Richtungen fließen zu lassen.

15. Verfahren zum Filtern einer ersten Flüssigkeit, die einen Analyten enthält, um eine gefilterte Flüssigkeit bereitzustellen, die weniger Analyt als die erste Flüssigkeit enthält, wobei das Verfahren die folgenden Schritte umfasst:
Einführen (1102) der ersten Flüssigkeit in einen Einlass eines Netzwerks von Kanälen mit einem oder mehreren ersten Kanälen, wobei jeder erste Kanal eine Höhe im Bereich von 50 Mikrometer bis 500 Mikrometer, eine Breite im Bereich von 50 Mikrometer bis 900 Mikrometer und eine Länge im Bereich von 3 Zentimeter bis 20 Zentimeter aufweist;
Einführen (1104) von Filtrat in einen Einlass mindestens eines zweiten Kanals, der zu dem einen oder den mehreren ersten Kanälen komplementär ist, wobei eine Filtrationsmembran (204; 404; 604) den einen oder die mehreren ersten Kanäle von dem mindestens einen zweiten Kanal trennt;
Fließenlassen (1106) des Filtrats durch mindestens ein Druckmerkmal in dem mindestens einen zweiten Kanal, wobei das mindestens eine Druckmerkmal konfiguriert ist, um in einem dem Druckmerkmal vorgelagerten Abschnitt des mindestens einen zweiten Kanals einen Hochdruck, in einem dem Druckmerkmal nachgelagerten Abschnitt des mindestens einen zweiten Kanals einen Niederdruck und entlang der Länge des mindestens einen zweiten Kanals ein nichtlineares Druckprofil zu ergeben, sodass mindestens ein Teil des Analyten der ersten Flüssigkeit von dem einen oder den mehreren ersten Kanälen durch die Filtrationsmembran und in den mindestens einen zweiten Kanal transportiert wird; und
Sammeln (1112) der gefilterten Flüssigkeit von einem Auslass eines oder mehrerer der ersten Kanäle, wobei der Auslass des einen oder der mehreren ersten Kanäle dem Einlass des mindestens einen entsprechenden zweiten Kanals entgegengesetzt angeordnet ist und ein Einlass des einen oder der mehreren ersten Kanäle einem Auslass des mindestens einen komplementären zweiten Kanals entgegengesetzt angeordnet ist.

## Revendications

1. Dispositif micro fluidique (100), comprenant :
un réseau de canaux ayant un ou plusieurs premiers canaux (208 ; 408 ; 608 ; 708), chaque premier canal ayant une hauteur dans la plage de 50 microns à 500 microns, une largeur dans la plage de 50 microns à 900 microns, et une longueur dans la plage de 3 centimètres à 20 centimètres ;
au moins un deuxième canal (210 ; 410 ; 610 ; 710) complémentaire d'un ou de plusieurs des premiers canaux, l'au moins un deuxième canal comprenant au moins un élément de pression (216 ; 416 ; 616 ; 754 ; 916) configuré pour produire une haute pression dans une partie (228) de l'au moins un deuxième canal en amont de l'élément de pression et une faible pression dans une partie (226) de l'au moins un deuxième canal en aval de l'élément de pressurisation ; et
une membrane de filtration (204 ; 404 ; 604) séparant les un ou plusieurs premiers canaux de l'au moins un deuxième canal ; dans lequel la partie en amont de l'au moins un deuxième canal est située à l'opposé d'une partie en aval des uns ou plusieurs premiers canaux complémentaires, de sorte que quand du fluide est amené à s'écouler à travers les premiers et deuxièmes canaux, un profil de pression non linéaire (318 ; 518) existe le long de la longueur de l'au moins un deuxième canal et un gradient de pression existe de part et d'autre de la membrane séparant les un ou plusieurs premiers canaux de l'au moins un deuxième canal complémentaire.

2. Dispositif micro fluidique de la revendication 1, dans lequel les un ou plusieurs premiers canaux (1008) comprennent au moins un deuxième élément de pression (1016) configuré pour produire une haute pression dans une partie des un ou plusieurs premiers canaux en amont de l'élément de pression et une basse pression dans une partie des un ou plusieurs premiers canaux en aval de l'élément de pression.

3. Dispositif micro fluidique de la revendication 1, dans lequel le profil de pression non linéaire le long de la longueur de l'au moins un deuxième canal est sensiblement une fonction échelon.

4. Dispositif micro fluidique de la revendication 1, dans lequel l'élément de pression comprend un des éléments suivants :
• une paroi partielle (416) configurée pour restreindre l'écoulement de fluide dans l'au moins un deuxième canal, produisant la haute pression au niveau d'une partie en amont de l'au moins un deuxième canal et la basse pression au niveau d'une partie en aval de l'au moins un deuxième canal ;
• une section (216) de l'au moins un deuxième canal qui rétrécit pour diminuer la section transversale d'une partie du canal, produisant la haute pression au niveau d'une partie en amont de l'au moins un deuxième canal et la basse pression au niveau d'une partie en aval de l'au moins un deuxième canal ;
• une section (754) de l'au moins un deuxième canal configurée pour diriger le fluide le long d'un parcours sinueux, produisant la haute pression au niveau d'une partie en amont de l'au moins un deuxième canal et la basse pression au niveau d'une partie en aval de l'au moins un deuxième canal ;
• au moins un élément parmi un tampon poreux (616), une deuxième membrane, et un gel inséré jusque dans l'au moins un deuxième canal, produisant la haute pression au niveau d'une partie en amont de l'au moins un deuxième canal et la basse pression au niveau d'une partie en aval de l'au moins un deuxième canal ;
• un dispositif qui extrait du travail d'un écoulement de fluide dans l'au moins un deuxième canal, fournissant ainsi la haute pression au niveau d'une partie en amont de l'au moins un deuxième canal et la basse pression au niveau d'une partie en aval de l'au moins un deuxième canal, dans lequel le dispositif est soit une pompe inversée soit une turbine située à l'intérieur de l'au moins un deuxième canal ; ou
• un dispositif d'étranglement (916) couplé à l'au moins un deuxième canal et configuré pour contrôler un écoulement de fluide dans l'au moins un deuxième canal, produisant ainsi la haute pression au niveau d'une partie en amont de l'au moins un deuxième canal et la basse pression au niveau d'une partie en aval de l'au moins un deuxième canal, dans lequel le dispositif d'étranglement inclut une soupape.

5. Dispositif micro fluidique de la revendication 1, dans lequel l'au moins un deuxième canal est formé à partir d'un matériau flexible, et dans lequel l'élément de pression constitue une partie de l'au moins un deuxième canal qui peut être comprimé pour réduire sa section transversale, produisant ainsi la haute pression au niveau d'une partie en amont de l'au moins un deuxième canal et la basse pression au niveau d'une partie en aval de l'au moins un deuxième canal.

6. Dispositif microfluidique de la revendication 1, dans lequel l'élément de pression peut être configuré pour fournir un profil de pression voulu dans l'au moins un deuxième canal.

7. Dispositif micro fluidique de la revendication 1, comprenant en outre au moins un élément parmi un capteur de débit (806a, 806b) et un capteur de pression (804a, 804b) pour déterminer au moins une valeur parmi un débit et une pression de fluide dans les un ou plusieurs premiers canaux et l'au moins un deuxième canal.

8. Dispositif micro fluidique de la revendication 7, comprenant en outre un processeur (802) configuré pour commander l'élément de pression en réponse au débit déterminé ou à la pression de fluide déterminée.

9. Dispositif micro fluidique de la revendication 1, dans lequel le dispositif est configuré pour maintenir une différence de pression maximale dans l'au moins un deuxième canal dans une plage de 200 mmHg à 2 000 mmHg.

10. Dispositif micro fluidique de la revendication 1, dans lequel la membrane a une épaisseur dans la plage de 5 µm à 300 µm.

11. Dispositif micro fluidique de la revendication 1, dans lequel la taille des pores de la membrane est sélectionnée pour permettre l'élimination de particules avec un poids moléculaire de pas plus d'environ 60 kDa.

12. Dispositif micro fluidique de la revendication 1, comprenant en outre un revêtement anticoagulant sur les surfaces internes des un ou plusieurs premiers canaux.

13. Dispositif micro fluidique de la revendication 1, dans lequel les un ou plusieurs premiers canaux sont configurés pour maintenir les taux de cisaillement à la paroi dans la plage de 200 secondes inverses à 2 000 secondes inverses quand le sang est transporté à travers les un ou plusieurs premiers canaux.

14. Dispositif micro fluidique de la revendication 1, dans lequel les un ou plusieurs premiers canaux et l'au moins un deuxième canal sont configurés pour amener le fluide à s'écouler dans des directions opposées.

15. Procédé de filtration d'un premier liquide contenant un analyte pour fournir un liquide filtré contenant moins d'analyte que le premier liquide, le procédé comprenant les étapes consistant à :
introduire (1102) le premier liquide jusque dans une admission d'un réseau de canaux ayant un ou plusieurs premiers canaux, chaque premier canal ayant une hauteur dans la plage de 50 microns à 500 microns, une largeur dans la plage de 50 microns à 900 microns, et une longueur dans la plage de 3 centimètres à 20 centimètres ;
introduire (1104) le filtrat jusque dans une admission d'au moins un deuxième canal complémentaire des un ou plusieurs premiers canaux, une membrane de filtration (204 ; 404 ; 604) séparant les un ou plusieurs premiers canaux de l'au moins un deuxième canal;
amener (1106) le filtrat à s'écouler à travers au moins un élément de pression dans l'au moins un deuxième canal, dans lequel l'au moins un élément de pression est configuré pour produire une haute pression dans une partie de l'au moins un deuxième canal en amont de l'élément de pression, une faible pression dans une partie de l'au moins un deuxième canal en aval de l'élément de pression, et un profil de pression non linéaire le long de l'au moins un deuxième canal, de sorte qu'au moins une certaine quantité de l'analyte du premier liquide est transportée des un ou plusieurs premiers canaux à travers la membrane de filtration et jusque dans l'au moins un deuxième canal ; et
collecter (1112) le liquide filtré depuis une évacuation d'un ou de plusieurs des premiers canaux, dans lequel l'évacuation des un ou plusieurs premiers canaux est située à l'opposé de l'admission de l'au moins un deuxième canal correspondant, et une admission des un ou plusieurs premiers canaux est située à l'opposé d'une évacuation de l'au moins un deuxième canal complémentaire.
